# EUROPÄISCHE PATENTSCHRIFT

(11) **EP 0 582 246 B1**
(45) Veröffentlichungstag und Bekanntmachung des Hinweises auf die Patenterteilung: **22.01.1997**
(21) Anmeldenummer: 93112340.0
(22) Anmeldetag: 02.08.1993
(51) Int. Cl.: C07C 69/33, C07C 67/08, C07C 67/58, C07C 67/56, C11D 1/66, C10M 129/74, A61K 7/50

(54) **Polyglycerinfettsäureestergemisch**
Mixture of polyglycerine fatty acids esters
Mélange d'esters d'acides gras et de polyglycérine

(30) Priorität: 07.08.1992 DE 4226174
(43) Veröffentlichungstag der Anmeldung: 09.02.1994
(73) Patentinhaber: Solvay Fluor und Derivate GmbH, D-30173 Hannover (DE)
(72) Erfinder: Jakobson, Gerald, D-4134 Rheinberg 2 (DE); Siemanowski, Werner, D-4134 Rheinberg 1 (DE); Uhlig, Karl-Heinz, D-4150 Krefeld-Traar (DE)
(74) Vertreter: Lauer, Dieter, Dr.

(56) Entgegenhaltungen:
- EP-A- 0 299 295
- EP-A- 0 499 700
- DE-A- 4 023 593

## Beschreibung

Die vorliegende Erfindung betrifft ein Polyglycerinfettsäureestergemisch aus Mono-, Di- und Polyestern von Polyglycerinen mit einem Polymerisationsgrad von 2 bis 8 und mindestens einer gesättigten und/oder ungesättigten Fettsäure. Sie betrifft weiterhin ein Badezusatzpräparat, enthaltend dieses Polyglycerinfettsäureestergemisch, sowie die Verwendung des Polyglycerinfettsäureestergemisches und ein Verfahren zu seiner Herstellung.

Es ist bereits bekannt, Polyglycerinfettsäureester und -estergemische wegen ihrer lipophilen und hydrophilen Eigenschaften als oberflächen- beziehungsweise grenzflächenaktive Verbindungen zur Zubereitung und Stabilisierung von O/W- und W/O-Systemen einzusetzen. Es handelt sich bei diesen Polyglycerinfettsäureestern um nichtdefinierte Partialester von Polyglycerinen mit gesättigten und/oder ungesättigten Fettsäuren, wobei zahlreiche Möglichkeiten zur Herstellung von Emulgatoren und Solubilisatoren mit geeignet abgestimmter Fett- bzw. Wasserlöslichkeit bestehen. Der HLB-Wert von Polyglycerinfettsäureestern oder -estergemischen hängt von ihrer Zusammensetzung ab. Diese wird durch den Syntheseweg bestimmt, insbesondere von der Art und dem Verhältnis der Reaktanten sowie von den Reaktionsbedingungen.

Die Herstellung von grenzflächenaktiven Verbindungen mit ganz bestimmten HLB-Werten durch Veresterung von Polyglycerinen mit Fettsäuren wirft jedoch verschiedene Probleme auf.

Einerseits sind die Reaktionsmechanismen hinsichtlich der Selektivität der Veresterung und der auftretenden Zahl an Substitutionen am Polyglyceringrundgerüst bis heute weitgehend ungeklärt, so daß die Aktivität der Polyglycerinfettsäureester als Emulgatoren und Solubilisatoren weder kontrolliert noch eindeutig vorhergesagt werden kann.

Insbesondere ist es daher schwierig, durch Veresterung von Gemischen verschiedener Glycerinoligomeren mit Fettsäuren Produkte mit ganz bestimmten grenzflächenaktiven Eigenschaften herzustellen, zumal derartige Gemische, bedingt durch die technischen Verfahren zu ihrer Herstellung, häufig aus einer Vielzahl von verschiedenen Polyglycerinen sowie einem mehr oder weniger hohen Anteil an cyclischen Glycerinoligomeren bestehen.

Andererseits werden die hydrophilen und lipophilen Eigenschaften der Polyglycerinfettsäureester durch die veresterten Fettsäuren, das Polyglyceringrundgerüst und den Veresterungsgrad bestimmt. Dabei sind insbesondere die zunehmende Hydrophilie der Polyglycerine mit steigendem Polymerisationsgrad, die spezifische Oberflächenaktivität der jeweils ausgewählten Fettsäuren und Fettsäuregemische als Veresterungsprodukt, ihre Löslichkeit im Polyglycerin sowie das ungewöhnlich starke Absinken des HLB-Wertes der Polyglycerinfettsäureester bzw. -estergemische bei Verwendung von Fettsäuren mit hohen Molmassen zu berücksichtigen. Außerdem ist das Veresterungsverhältnis von den Gewichts- bzw. Molverhältnissen der eingesetzten Reaktanten, Polyglycerin und Fettsäure, zueinander abhängig.

Im Stand der Technik werden Polyglycerinfettsäureester mit definierten Eigenschaften bisher durch gezielte Veresterung ganz bestimmter Glycerinoligomeren, wie beispielsweise Triglycerin oder Tetraglycerin, vorzugsweise unter Einsatz von Schutzgruppen, hergestellt.

Der Einsatz von Polyglyceringemischen als Veresterungsedukt ist beispielsweise aus der DE-OS 40 23 593 bekannt. Hier wird eine Polyglycerinmischung verestert, die nicht nur eine breite Verteilung hinsichtlich der in ihr enthaltenen Glycerinoligomeren aufweist, sondern die auch, bedingt durch das Herstellungsverfahren des Gemisches, einen mehr oder weniger hohen Anteil an cyclischen Polyglycerinen enthält. Das Veresterungsprodukt gemäß DE-OS 40 23 593 stellt ein nichtflüssiges Tensid dar, dessen Eigenschaften hinsichtlich seiner Wirkung als Öl-in-Wasser-Emulgator, insbesondere in Bezug auf die erreichten HLB-Werte (Hydrophilic-Lipophilic-Balance) und die Stabilität der hergestellten Emulsionen, unbefriedigend sind.

Aufgabe der vorliegenden Erfindung war es daher, ein Polyglycerinfettsäureestergemisch bereitzustellen, das abweichend vom Stand der Technik einen flüssigen, wasserlöslichen Emulgator zur Herstellung von stabilen Öl-in-Wasser-Systemen darstellt, wobei das eingesetzte Polyglycerin eine enge Verteilung hinsichtlich der in ihm enthaltenen Glycerinoligomeren und keine oder nur geringe Mengen an cyclischen Komponenten aufweisen soll.

Insbesondere sollte auch ein Polyglycerinfettsäureestergemisch gefunden werden, das sich sehr gut zur Emulgierung und/oder Solubilisierung von ätherischen, mineralischen sowie fetten tierischen und pflanzlichen Ölen eignet, was Schwierigkeiten bereitet, weil derartige Öle oder Ölgemische naturgemäß nur schwer zu emulgieren bzw. solubilisieren sind. Polyglycerinfettsäureester oder -estergemische konnten auf diesem Gebiet bisher nur unbefriedigend eingesetzt werden.

Weiterhin sollte das Polyglycerinfettsäureestergemisch neben einer hohen Hydrophilie auch vorteilhafte dermatologische und toxikologische Eigenschaften aufweisen, so daß es in kosmetischen und pharmazeutischen Zubereitungen zur Einstellung bestimmter hautpflegender Effekte dienen kann.

Es wurde nun gefunden, daß ein Polyglycerinfettsäureestergemisch, das (bezogen auf 100 Gewichtsteile des Polyglycerinfettsäureestergemisches) einen Anteil von
- 20 bis 65 Gew.-%: Triglycerinfettsäureestern,
- 20 bis 50 Gew.-%: Tetraglycerinfettsäureestern und
- 5 bis 40 Gew.-%: höheren Polyglycerinfettsäureestern
aufweist und keine oder nur geringe Mengen (weniger als 5 Gew.-%) an Diglycerinfettsäureestern enthält, wobei die Fettsäurekomponente aus einer oder mehreren Fettsäuren, ausgewählt aus den gesättigten und/oder ungesättigten, verzweigten und/oder unverzweigten C₆- bis C₁₄-Fettsäuren, mit einem Anteil unter 10 Gew.-% an Fettsäuren mit mehr als 14 C-Atomen, besteht und der HLB-Wert des Polyglycerinfettsäureestergemisches größer als 8 ist,
alle vorteilhaften Eigenschaften, die in der Aufgabenstellung genannt wurden, aufweist.

Es wurde weiterhin gefunden, daß das erfindungsgemäße flüssige Polyglycerinfettsäureestergemisch nicht nur ausgezeichnet zur Emulgierung und Solubilisierung von Ölen und ölartigen Verbindungen oder Zusammensetzungen, insbesondere ätherischen und fetten pflanzlichen oder tierischen Ölen, geeignet ist, sondern überraschenderweise auch eine Spontanemulgierung der vorgenannten Öle, insbesondere auch in verdünnten wäßrigen Lösungen, bewirkt. Die Vermischung dieser Öle mit dem erfindungsgemäßen Polyglycerinfettsäureestergemisch ergibt optisch klare Flüssigkeiten, da die ölige Verbindung in hohem Maße solubilisiert wird.

Das erfindungsgemäße Polyglycerinfettsäureestergemisch kann aufgrund seiner hohen Hydrophilie als Lösungs- und Emulgiermittel, Lösungsvermittler, Netz- und Dispergiermittel zur Herstellung stabiler O/W-Systeme verwendet werden. Produkte, insbesondere kosmetische und pharmazeutische Zubereitungen, die das erfindungsgemäße Polyglycerinfettsäureestergemisch als Tensid enthalten, sind bei den üblichen Testtemperaturen zeitlich unbegrenzt stabil und bereiten somit keine Lager- und Transportprobleme. Geringe Säure- oder Salzkonzentrationen können das eingestellte Phasengleichgewicht ebenfalls nicht stören: beispielsweise treten in einer Mischung aus 1 Gewichtsteil des erfindungsgemäßen Polyglycerinfettsäureestergemisches und 100 Gewichtsteilen einer 10 gew.-%igen Kochsalzlösung keine Trübungen bzw. Ausscheidungen auf. Das erfindungsgemäße Polyglycerinfettsäureestergemisch ist daher mit einer Vielzahl von Stoffen kombinierbar und kann einen breiten Anwendungsbereich finden.

Gegenüber den häufig als Emulgier-, Solubilisierungs- und Netzmittel eingesetzten Äthoxylaten weist das erfindungsgemäße Polyglycerinfettsäureestergemisch eine bessere Bioabbaubarkeit auf und kann zudem aufgrund seiner vorteilhaften toxikologischen und dermatologischen Eigenschaften problemlos in kosmetische und pharmazeutische Zubereitungen sowie Nahrungsmittel eingearbeitet werden.

Das erfindungsgemäße Polyglycerinfettsäureestergemisch besitzt darüber hinaus eine hohe Hautpflegewirkung und insbesondere ein hohes Rückfettungsvermögen.

Nach einer bevorzugten Ausführungsform weist das erfindungsgemäße Polyglycerinfettsäureestergemisch (bezogen auf 100 Gewichtsteile des Polyglycerinfettsäureestergemisches) einen Anteil von
- 22 bis 32 Gew.-%: Triglycerinfettsäureestern,
- 39 bis 49 Gew.-%: Tetraglycerinfettsäureestern und
- 24 bis 34 Gew.-%: höheren Polyglycerinfettsäureestern
auf und enthält keine oder nur geringe Mengen (weniger als 3 Gew.-%) an Diglycerinfettsäureestern, wobei die Fettsäurekomponente aus einer oder mehreren Fettsäuren, ausgewählt aus den gesättigten und/oder ungesättigten, verzweigten und/oder unverzweigten C₈- bis C₁₂-Fettsäuren, mit einem Anteil unter 5 Gew.-% an Fettsäuren mit mehr als 14 C-Atomen, besteht und der HLB-Wert des Polyglycerinfettsäureestergemisches größer als 10 ist.

Nach einer bevorzugten Ausführungsform weist das erfindungsgemäße Polyglycerinfettsäureestergemisch einen Anteil von 5 bis 20 Gewichtsprozent an Pentaglycerinfettsäureestern auf.

Als höhere Polyglycerinfettsäureester werden im Rahmen der vorliegenden Erfindung solche Derivate von Glycerinoligomeren bezeichnet, die 5 bis 8 Glycerineinheiten im Molekül aufweisen, also einen Polymerisationsgrad von 5 bis 8 besitzen.

Als Fettsäuren in dem erfindungsgemäßen Polyglycerinfettsäureestergemisch eignen sich alle gesättigten und ungesättigten, verzeigten und unverzweigten C₆- bis C₁₄-Fettsäuren, allein oder in Mischungen untereinander. Dabei hat sich herausgestellt, daß die untere Grenze in der C-Atomzahl durch diejenige Fettsäure festgelegt wird, die mit der kürzesten Kette gerade noch Oberflächenaktivität als Vereesterungsprodukt zeigt. Da sich Fettsäuren mit C₁₅ und höheren C-Ketten schlecht im Polyglycerin lösen, ist die obere Grenze der erfindungsgemäß ausgewählten Fettsäuren durch solche mit einer maximalen C-Atomzahl von etwa 14 festgelegt.

Es hat sich jedoch gezeigt, daß geringe Anteile an Fettsäuren mit C₁₅ und höheren C-Ketten die besonderen grenzflächenaktiven Eigenschaften des erfindungsgemäßen Polyglycerinfettsäureestergemisches nicht stören, weshalb die erfindungsgemäß ausgewählten Fettsäuren einen Gehalt von unter 10 Gewichtsprozent, vorzugsweise unter 5 Gewichtsprozent, an Fettsäuren mit mehr als 14 C-Atomen aufweisen können, wobei hierdurch auch bestimmte zusätzliche Eigenschaften in dem erfindungsgemäßen Polyglycerinfettsäureestergemisch eingestellt werden können.

Bevorzugt besteht die Fettsäurekomponente aus Caprylsäure, caprinsäure, Laurinsäure, Undecensäure, 2-Ethylhexansäure und/oder Kokosfettsäure. Insbesondere diese Fettsäuren dienen der Einstellung bestimmter zusätzlicher Eigenschaften, wie hautpflegender oder antimykotischer Wirkungen, in dem erfindungsgemäßen Polyglycerinfettsäureestergemisch.

Weiterhin werden besonders vorteilhafte Eigenschaften hinsichtlich Pflegewirkung und Grenzflächenaktivität erreicht, wenn das erfindungsgemäße Polyglycerinfettsäureestergemisch zu mehr als 50 Gewichtsprozent aus Mono-und Diestern besteht.

Die vorliegende Erfindung betrifft weiterhin ein Badezusatzpräparat, insbesondere ein Ölbadpräparat, in dem die vorteilhaften Wirkungen des erfindunggemäßen Polyglycerinfettsäureestergemisches besonders zur Geltung kommen.

Das erfindungsgemäße Badezusatzpräparat, insbesondere Ölbadpräparat enthält ein Polyglycerinfettsäureestergemisch aus Mono-, Di- und Polyestern von Polyglycerinen mit einem Polymerisationsgrad von 2 bis 8 und mindestens einer gesättigten und/oder ungesättigten Fettsäure, mindestens eine ölige oder ölhaltige Komponente, ausgewählt aus den natürlichen und synthetischen, mineralischen, ätherischen und fetten tierischen und pflanzlichen Ölen, gegebenenfalls ein Lösemittel oder Lösemittelgemisch sowie gegebenenfalls weitere Hilfs- und Zusatzstoffe und ist dadurch gekennzeichnet, daß es
- 10 bis 60 Gew.-%: eines Polyglycerinfettsäureestergemisches als wasserlöslichen Emulgator und/oder Solubilisator,
- 10 bis 60 Gew.-%: eines kosmetisch und/oder therapeutisch wirkenden Öls, Ölgemisches und/oder Ölkomponente und
- 0 bis 70 Gew.-%: Wasser
enthält, wobei das Polyglycerinfettsäureestergemisch (bezogen auf 100 Gewichtsteile des Polyglycerinfettsäureestergemisches) einen Anteil von
- 20 bis 65 Gew.-%: Triglycerinfettsäureestern,
- 20 bis 50 Gew.-%: Tetraglycerinfettsäureestern und
- 5 bis 40 Gew.-%: höheren Polyglycerinfettsäureestern
aufweist und keine oder nur geringe Mengen (weniger als 5 Gew.-%) an Diglycerinfettsäureestern enthält und die Fettsäurekomponente aus einer oder mehreren Fettsäuren, ausgewählt aus den gesättigten und/oder ungesättigten, verzweigten und/oder unverzweigten C₆- bis C₁₄-Fettsäuren, mit einem Anteil unter 10 Gew.-% an Fettsäuren mit mehr als 14 C-Atomen, besteht,

Die hervorragende emulgierende und/oder solubilisierende Wirkung des erfindungsgemäßen Polyglycerinfettsäureestergemisches erweist sich vorzugsweise auch in Badezusatzpräparaten, die ölige oder ölhaltige Substanzen enthalten, welche naturgemäß schwer zu emulgieren bzw. solubilisieren sind, als sehr vorteilhaft. Das ausgezeichnete Spontanemulgierungsvermögen des erfindungsgemäßen Polyglycerinfettsäureestergemisches zeigt sich insbesondere bei Einarbeitung des erfindungsgemäßen Badezusatzpräparates in stark verdünnte wäßrige Lösungen, wie beispielsweise ein Badewasser, wobei die ölige oder ölhaltige Komponente im Badewasser und/oder auf der Wasseroberfläche (Spreitung) feinverteilt wird, so daß sie ihre kosmetische und/oder therapeutische Wirkung optimal entfalten kann.

Das erfindungsgemäße Badezusatzpräparat ist nicht nur physiologisch unbedenklich und biologisch leicht abbaubar, sondern es weist auch einen hautpflegenden und -schützenden, insbesondere einen rückfettenden Effekt auf, der auch bei großer Verdünnung des erfindungsgemäßen Badezusatzpräparates in einem Badewasser zu einem angenehmen Hautgefühl nach dem Bad führt und der Austrockung der Haut durch das Badewasser entgegenwirkt.

Entsprechend der spezifischen Auswahl des kosmetisch und/oder therapeutisch wirkenden Öls, Ölgemisches und/oder Ölkomponente werden besondere Pflegeeigenschaften und/oder medizinisch-therapeutische bzw. pharmakologische Effekte, wie beispielsweise heilende, lindernde, beruhigende, entspannende, regenerierende und/oder vitalisierende physiologische Wirkungen, in dem erfindungsgemäßen Badezusatzpräparat eingestellt.

Gemäß einer vorzugsweisen Ausführungsform enthält das erfindungsgemäße Badezusatzpräparat
- 15 bis 50 Gew.-%: eines Polyglycerinfettsäureestergemisches,
- 15 bis 50 Gew.-%: eines kosmetisch und/oder therapeutisch wirkenden Öls, Ölgemisches und/oder Ölkomponente und
- 0,5 bis 60 Gew.-%: Wasser,
wobei das Polyglycerinfettsäureestergemisch (bezogen auf 100 Gewichtsteile des Polyglycerinfettsäureestergemisches) einen Anteil von
- 22 bis 32 Gew.-%: Triglycerinfettsäureestern,
- 39 bis 49 Gew.-%: Tetraglycerinfettsäureestern und
- 24 bis 34 Gew.-%: höheren Polyglycerinfettsäureestern
aufweist und keine oder nur geringe Mengen (weniger als 3 Gew.-%) an Diglycerinfettsäureestern enthält und die Fettsäurekomponente aus einer oder mehreren Fettsäuren, ausgewählt aus den gesättigten und/oder ungesättigten, verzweigten und/oder unverzweigten C₈- bis C₁₂-Fettsäuren, mit einem Anteil unter 5 Gew.-% an Fettsäuren mit mehr als 14 C-Atomen, besteht.

Badezusatzpräparate gemäß der Erfindung enthalten als Fettsäurekomponente in dem erfindungsgemäßen Polyglycerinfettsäureestergemisch vorzugsweise Caprylsäure, Caprinsäure, Laurinsäure, Undecensäure, 2-Ethylhexansäure oder Kokosfettsäure, wobei diese Fettsäuren auch als Gemische untereinander vorliegen können. Durch die vorgenannten Verbindungen können in dem erfindungsgemäßen Polyglycerinfettsäureestergemisch zusätzliche bzw. besondere Eigenschaften eingestellt werden. Dies sind beispielsweise entweder vorteilhafte hautpflegende Eigenschaften oder, wie im Fall der Undecensäure, eine antimykotische Wirkung, was für die Konservierung des Badezusatzpräparates von Vorteil ist.

Als kosmetisch wirkendes Öl enthält das erfindungsgemäße Badezusatzpräparat vorzugsweise fette pflanzliche Öle, wie Jojobaöl, Sojaöl, Sesamöl, Erdnußöl, Sonnenblumenöl, Olivenöl, Palmöl, Palmkernöl, Rizinusöl, Kakaoöl, Kokosnußöl, Mandelöl oder Weizenkeimöl, wobei diese Öle allein oder in Mischungen untereinander eingesetzt werden.

Des weiteren kann das erfindungsgemäße Badezusatzpräparat kosmetisch wirkende tierische Öle enthalten, vorzugsweise synthetisches Bürzeldrüsenöl, das einen hydrophobierenden Effekt auf der Haut bewirkt und das durch das ausgezeichnete Solubilisierungvermögen des erfindungsgemäßen Polyglycerinfettsäureestergemisches ohne Bildung von Ausscheidungen in das erfindungsgemäße Badezusatzpräparat eingearbeitet werden kann.

Besondere pharmakologische bzw. medizinisch-therapeutische Eigenschaften werden in dem erfindungsgemäßen Badezusatzpräparat durch entsprechend wirkende Öle oder Ölgemische eingestellt, vorzugsweise durch Zusatz von ätherischen Ölen, wie Rosmarinöl, Lavendelöl, Melissenöl, Salbeiöl, Knoblauchöl, Wacholderbeeröl, Anisöl, Kardamonöl, Pimentöl, Anisöl, Kümmelöl, Zitronenöl, Orangenöl, Pfefferminzöl, Campferöl, Nelkenöl, Fichtennadelöl oder Eukalyptusöl, wobei diese Öle allein oder in Mischungen untereinander, eingesetzt werden können.

Weiterhin kann das erfindungsgemäße Badezusatzpräparat als kosmetisch wirkendes Öl und/oder Ölkomponente natürliche oder synthetische Verbindungen, wie vorzugsweise Isopropylmyristat, Isopropylpalmitat, Ölsäuredecylester, 2-Octyldodecanol, Cetyl-Stearylisononanoat, Lanolin- oder Cholesterinderivate sowie Capryl-caprinsäuretriglycerid, enthalten, wobei diese Verbindungen allein oder in Mischungen untereinander eingesetzt werden und ebenfalls eine hautpflegende und/oder rückfettende Wirkung haben.

Die vorgenannten natürlichen oder synthetischen, fetten pflanzlichen und tierischen Öle sowie ätherischen Öle und ölkomponenten können sich gegenseitig ganz oder teilweise in dem erfindungsgemäßen Badezusatzpräparat ersetzen. Sie können aber auch durch pharmazeutisch bzw. therapeutisch wirkende Mineralöle oder -ölgemische, wie beispielsweise Paraffinöl, ganz oder teilweise in dem erfindungsgemäßen Badezusatzpräparat ersetzt sein.

Ein zusätzlicher parfümierender Effekt kann in dem erfindungsgemäßen Badezusatzpräparat durch die Zugabe bestimmter ätherischer Öle, vorzugsweise Rosenöl, Jasminöl, Veilchenöl, Mimosenöl, Orangenöl, Neroliöl, Patchouliöl, Sandelholzöl oder Zimtöl sowie durch Zusatz synthetischer oder natürlicher Parfümkompositionen eingestellt werden, wobei diese Öle allein oder in Mischungen untereinander eingesetzt werden. Man erhält dann ein sogenanntes Duftbad.

Als weitere therapeutisch wirksame Substanzen können Lösungen pflanzlicher Extrakte, wie solche der Kamille, in dem Badezusatzpräparat enthalten sein, um beispielsweise entzündliche Prozesse auf der Haut und in den Atemwegsorganen zu lindern bzw. zu heilen.

Das erfindungsgemäße Badezusatzpräparat kann auch als Schaumbad formuliert werden. Hierzu werden zusätzlich schaumaktive Mittel eingearbeitet, wie beispielsweise anionische Tenside, vorzugsweise Alkylethersulfonate und -sulfate, insbesondere Natriumlaurylethersulfat, um ein gutes Schäumvermögen auch bei Fettbelastung zu erhalten.

Als Lösungsmittel kann das erfindungsgemäße Badezusatzpräparat Wasser, vorzugsweise entmineralisiertes Wasser, enthalten, das gegebenenfalls geringe Mengen an wasserlöslichen organischen Lösungsmitteln, die gesundheitlich unbedenklich sind, aufweist. Dies sind beispielsweise Glycerin und/oder niedere Alkohole, wie 1,2-Propandiol, die als zusätzliche Lösungsvermittler eingesetzt werden können und Trübungen durch Ausflockung von organischen Bestandteilen in dem Badezusatzpräparat verhindern.

Der Einsatz geringer Wassermengen als Lösungsmittel ist insbesondere dann vorteilhaft, wenn das erfindungsgemäße Badezusatzpräparat aus emulgierendem bzw. solubilisierendem Polyglycerinfettsäureestergemisch und der öligen oder ölhaltigen Komponente eine trübe Lösung bildet. Durch den Wasserzusatz erhält man dann eine optisch klare Lösung.

Inbesondere hinsichtlich seines ausgezeichneten Solubilisierungsvermögens in wasserhaltigen Ölbadpräparaten zeigt das erfindungsgemäße Polyglycerinfettsäureestergemisch seine Überlegenheit gegenüber handelsüblichen Produkten.

Das erfindungsgemäße Polyglycerinfettsäureestergemisch kann zusätzlich mit einem weiteren Polyglycerinfettsäureestergemisch als Lösungsvermittler kombiniert werden. Hierzu eignet sich insbesondere ein Polyglycerincaprinat oder ein Polyglycerincocoat, die vorzugsweise gemäß der eigenen Patentanmeldung DE 41 05305 A hergestellt sind.

Bei einem höheren Anteil an pflanzlichen Ölen, insbesondere in einer wasserhaltigen Zubereitung, kann es vorteilhaft sein, das erfindungsgemäße Badezusatzpräparat gegen mikrobiellen Verderb zu schützen. Als bakteriostatische oder bakterizide Konservierungsmittel können Verbindungen wie Benzoate, Benzoesäurederivate, Sorbate, mikrobiologisch wirksame Phenole, wie 2,6-Di-tert.-butyl-methyl-phenol, und Dioxane, wie 5-Brom-5-nitro-1,3-dioxan, eingesetzt werden.

Gegen eine oxidative Zersetzung kann das erfindungsgemäße Badezusatzpräparat mit Antioxidationsmitteln, wie Tocopherolen, insbesondere Vitamin E, und/oder Butylhydroxytoluol, ausgestattet sein.

Die Konservierungsmittel werden gemäß üblichen Mengen in dem erfindungsgemäßen Badezusatzpräparat eingesetzt.

Als weitere Zusatz- oder Hilfsstoffe können pH-Wert-Regulatoren, Verdicker bzw. Viskositätsregulierungsmittel, wie Polyglykole, Propylenglykol, Ethanol, Isopropanol, Natriumpolyacrylat und/oder anorganische Salze, vorzugsweise Natriumchlorid, Komplexierungsmittel zur Maskierung von Metallionen, Feuchthaltemittel, wie Diglycerin, und Farbstoffe in dem erfindungsgemäßen Badezusatzpräparat enthalten sein.

Das erfindungsgemäße Badezusatzpräparat entfaltet seine vorteilhafte Wirkung insbesondere in einer Menge von 15 bis 30 ml auf etwa 200 Liter Badewasser.

Die Herstellung des erfindungsgemäßen Badezusatzpräparates erfolgt durch einfaches Vermischen der Bestandteile in entsprechenden Mischvorrichtungen, wobei die Reihenfolge der Zugabe der einzelnen Komponenten beliebig ist.

Die vorliegende Erfindung betrifft außerdem ein Verfahren zur Herstellung eines Polyglycerinfettsäureestergemisches aus Mono-, Di- und Polyestern von Polyglycerinen mit einem Polymerisationsgrad von 2 bis 8 und mindestens einer gesättigten und/oder ungesättigten Fettsäure, wobei das Verfahren dadurch gekennzeichnet ist, daß ein Polyglycerin, das (bezogen auf 100 Gew.-Teile Polyglycerin) einen Anteil von
- 20 bis 65 Gew.-%: Triglycerin,
- 20 bis 50 Gew.-%: Tetraglycerin und
- 5 bis 40 Gew.-%: höheren Polyglycerinen
aufweist und keine oder nur geringe Mengen (weniger als 5 Gew.-%) an Diglycerin enthält, mit einer oder mehreren Fettsäuren, ausgewählt aus den gesättigten und oder ungesättigten, verzeigten und/oder unverzeigten C₆- bis C₁₄-Fettsäuren, wobei die Fettsäure oder das Fettsäuregemisch einen Anteil von unter 10 Gew.-% an Fettsäuren mit mehr als 14 C-Atomen aufweist, in einem Molverhältnis des Polyglycerins zu der Fettsäure oder dem Fettsäuregemisch von
- 4 : 1 bis 1 : 1,: vorzugsweise
- 2,5 : 1 bis 1,5 : 1:
in Gegenwart mindestens eines Katalysators, vorzugsweise eines sauren Katalysators, und bei Unterdruck umgesetzt wird, indem das Reaktionsgemisch zunächst bis auf 140°C, vorzugsweise 145°C, aufgeheizt und der Druck bis auf 600 mbar, vorzugsweise 500 mbar, reduziert wird und dann das Reaktionsgemisch in einem Temperaturbereich von
- 140 bis 220°C,: vorzugsweise
- 145 bis 190°C,:
stufenweise oder kontinuierlich, über ein Temperaturprogramm gesteuert, erhitzt und dabei der Druck stufenweise oder kontinuierlich, über ein Druckprogramm gesteuert,
- von 600 auf 10 mbar,: vorzugsweise
- von 500 auf 20 mbar,:
erniedrigt wird, wobei das entstehende Reaktionswasser kontinuierlich abdestilliert und bei Erreichen einer Säurezahl von < 3 das erhaltene Polyglycerinfettsäureestergemisch abgekühlt und gegebenenfalls aufgearbeitet und/oder gereinigt wird.

Besonders bevorzugt wird in dem erfindungsgemäßen Verfahren ein Molverhältnis des Polyglycerins zu der Fettsäure oder dem Fettsäuregemisch von 2 : 1 eingestellt.

Das erfindungsgemäße Verfahren erlaubt die Herstellung von Polyglycerinfettsäureestergemischen definierter Hydrophilie durch eine einfache und kostengünstige Reaktionsführung, wobei eine gleichbleibende Qualität und reproduzierbare Zusammensetzung des Polyglycerinfettsäureestergemisches gewährleistet ist.

Nach einer bevorzugten Ausführungsform des erfindungsgemäßen Verfahrens wird ein Polyglycerin eingesetzt, das (bezogen auf 100 Gew.-teile Polyglycerin) einen Anteil von
- 22 bis 32 Gew.-%: Triglycerin,
- 39 bis 49 Gew.-%: Tetraglycerin und
- 24 bis 34 Gew.-%: höheren Polyglycerinen
aufweist und keine oder nur geringe Mengen (weniger als 3 Gew.-%) an Diglycerin enthält.

Als "Polyglycerin" wird ganz allgemein ein Gemisch von Polyglycerinen bzw. Glycerinoligomeren verschiedener Kettenlänge bezeichnet.

Das erfindungsgemäß eingesetzte Polyglycerin bzw. Polyglyceringemisch wird vorzugweise aus dem gemäß Patent DE 37 21 003 oder gemäß der DE-OS 34 10 520 erhaltenen Produktgemisch (Polyglycerin) hergestellt, indem diese Polyglyceringemische destillativ aufarbeitet werden, wobei insbesondere Diglycerin abgetrennt wird.

Nach einer vorzugsweisen Ausführungsform wird in dem erfindungsgemäßen Verfahren eine Fettsäure oder ein Fettsäuregemisch, ausgewählt aus den gesättigten und/oder ungesättigten, verzeigten und/oder unverzeigten C₈- bis C₁₂-Fettsäuren, mit einem Anteil unter 5 Gew-% an Fettsäuren mit mehr als 14 C-Atomen, eingesetzt.

Nach einer weiteren bevorzugten Ausführungsform des erfindungsgemäßen Verfahrens erfolgt eine Reinigung und/oder Aufarbeitung des hergestellten Polyglycerinfettsäureestergemisches, um nicht umgesetzte Polyglycerinanteile zumindest teilweise aus dem Verfahrensprodukt zu entfernen, was für bestimmte Anwendungen von Vorteil oder notwendig ist.

Hierzu wird das nach dem Verfahren gemäß einem oder mehreren der Ansprüche 8 bis 10 erhaltene Polyglycerinfettsäureestergemisch auf 30 bis 110°C, vorzugsweise 60 bis 80°C, abgekühlt und nachfolgend einer Extraktionsbehandlung unterworfen, indem das Polyglycerinfettsäureestergemisch mit einem organisch-chemischen Lösungsmittel oder Lösemittelgemisch versetzt und anschließend mit Wasser in vorzugsweise einer Extraktionsstufe Polyglycerin extrahiert wird, wobei in der ersten Extraktionsstufe zusätzlich eine der Säurezahl des Polyglycerinfettsäureestergemisches entsprechende und/oder der Katalysatormenge mindestens äquivalente Gewichtsmenge einer anorganischen und/oder organischen, basisch reagierenden Verbindung zugesetzt wird, und die nach der Extraktion verbleibende organische Phase von dem eingesetzten organischen Lösungsmittel und dem Restwassergehalt durch Destillation, vorzugsweise Vakuumdestillation oder Vakuumverdampfung, befreit wird.

Das in der Extraktionsbehandlung eingesetzte organischchemische Lösungsmittel oder Lösemittelgemisch weist vorzugsweise ein Wasseraufnahmevermögen von weniger als 30 Gew.-%, vorzugsweise weniger als 20 Gew.-% (bezogen auf 100 Gew.-Teile des organisch-chemischen Lösemittels oder Lösemittelgemisches), auf und/oder bildet mit Wasser bei der Destillation oder in der Gasphase ein azeotropes Gemisch.

Ein organisch-chemisches Lösemittel, das diese Eigenschaften aufweist, ist Ethylacetat, das vorzugsweise als mit Wasser gesättigte organische Phase in dem Extraktionsverfahren eingesetzt wird.

Weitere, erfindungsgemäß verwendbare organisch-chemische Lösemittel sind u. a. Butanol und/oder Toluol.

Die Neutralisation des Katalysators wird erfindungsgemäß vorzugsweise mit Natriumhydroxid, insbesondere mit wäßriger Natriumhydroxidlösung durchgeführt, wobei die alkalische Verbindung oder Lösung in der ersten Extraktionsstufe dem organisch-chemischen Lösemittel oder Lösemittelgemisch und/oder der Wasserphase zugegeben wird.

Als weitere Neutralisationsmittel sind Alkalicarbonate, vorzugsweise Natriumcarbonat und/oder Kaliumcarbonat, und/oder ein basischer Ionenaustauscher geeignet, die jeweils in der ersten Extraktionsstufe dem organischchemischen Lösemittel oder Lösemittelgemisch und/oder der Wasserphase zugegeben werden.

Laßt man das erfindungsgemäß hergestellte Polyglycerinfettsäureestergemisch vor einer Weiterverarbeitung und/oder Reinigung mehr als 0,5, vorzugsweise 1 bis 10 Stunden, stehen, so können aus dem Produktgemisch nicht umgesetzte Anteile an Polyglycerin gegebenenfalls ausgeschieden und abgetrennt werden.

Die gleichbleibende Qualität des Endproduktes, die definierte Zusammensetzung des Estergemisches und die Reproduzierbarkeit des Reaktionsergebnisses werden bei diesem Verfahren insbesondere durch die kontrollierte Reaktionsführung hinsichtlich Aufheizung und gleichzeitiger Druckerniedrigung in dem Reaktionsgemisch sowie durch die exakte Einhaltung bestimmter Druck- und Temperaturbereiche erhalten.

Die Reaktionsführung des erfindungsgemäßen Verfahrens kann kontinuierlich, über ein entsprechendes Temperatur- und Druckprogramm gesteuert, erfolgen, oder diskontinuierlich durch stufenweise Erwärmung und Druckverminderung.

Dabei wurde als vorteilhafte Ausführungsform des erfindungsgemäßen Verfahren gefunden, die Aufheizung des Reaktionsgemisches im Temperaturbereich von 140 bis 220°C, vorzugsweise 145 bis 190°C, und die Druckerniedrigung von 600 auf 10 mbar, vorzugsweise von 500 auf 20 mbar, in einem Zeitintervall von
- 2 bis 6 Stunden,: vorzugsweise
- 3 bis 4 Stunden,:
durchzuführen.

Gemäß einer weiteren bevorzugten Ausführungsform des erfindungsgemäßen Verfahrens erfolgt die Aufheizung des Reaktionsgemisches im Temperaturbereich von 140 bis 220°C, vorzugsweise 145 bis 190°C, stufenweise über
- 3 bis 6 Stufen,: vorzugsweise
- 4 bis 5 Stufen.:
Damit korrespondierend kann die Erniedrigung des Druckes von 600 auf 10 mbar, vorzugsweise 500 auf 20 mbar, schrittweise in einer bestimmten Anzahl von Stufen vorgenommen werden, vorzugsweise über
- 3 bis 6 Stufen,: insbesondere
- 4 bis 5 Stufen.:

Die Veresterungsreaktion nach dem erfindungsgemäßen Verfahren wird in Gegenwart eines Katalysators durchgeführt, vorzugsweise in Gegenwart einer sauren, sulfonsäuregruppenhaltigen Verbindung, wie beispielsweise Dodecylbenzolsulfonsäure oder andere Alkylbenzolsulfonsäuren.

Nach einer Ausführungsform wird dieser saure Katalysator in Kombination mit einer zweiten sauren, katalytisch und reduzierend wirkenden Verbindung eingesetzt. Eine in dieser Hinsicht geeignete Verbindung ist hypophosphorige Säure.

Gemäß einer weiteren vorteilhaften Ausführungsform des erfindungsgemäßen Verfahrens wird das Reaktionsgemisch in Gegenwart eines inerten Gases, vorzugsweise Stickstoff, umgesetzt.

Die Erfindung betrifft weiterhin die Verwendung des Polyglycerinfettsäureestergemisches nach einem oder mehreren der Ansprüche 1 bis 5 als Emulgator, Solubilisierungsmittel, Dispergierungmittel, Netzmittel und/oder Rückfetter in kosmetischen, pharmazeutischen oder chemisch-technischen Zubereitungen.

Das erfindungsgemäße Polyglycerinfettsäureestergemisch ist vielseitig verwendbar. Vorzugsweise dient es zur Emulgierung bzw. Dispergierung und/oder Solubilisierung von natürlichen oder synthetischen, ätherischen, mineralischen, fetten tierischen oder pflanzlichen ölen. Es eignet sich insbesondere ausgezeichnet zur Herstellung von ölhaltigen Badezusätzen, vorzugsweise Badeölen bzw. ölbädern.

Des weiteren wird das erfindungsgemäße Polyglycerinfettsäureestergemisch als Hautpflegeadditiv und/oder Wasch-, Reinigungs- oder Körperreinigungsmittel, Duschgel oder Duschmittel, Schaumbademittel, flüssiges Handreinigungsmittel oder Haarshampoo verwendet, da es neben der oberflächenaktiven Eigenschaft auch eine milde Reinigungswir-kung sowie einen Rückfettungseffekt besitzt und ein ange-nehmes Hautgefühl während und nach dem Reinigungsvorgang bewirkt.

Weitere Einsatzbereiche für das erfindungsgemäße Polyglycerinfettsäureestergemisch sind Lebensmittel sowie Arzneimittel, Salben, pharmazeutische und kosmetische Zubereitungen jeglicher Art, wo das Polyglycerinfettsäureestergemisch als Solübilisierungsmittel und/oder Emulgator bzw. Dispergiermittel und/oder Netzmittel wirkt. Seine vielseitige Verwendbarkeit ergibt sich u.a. dadurch, daß das erfindungsgemäße Polyglycerinfettsäureestergemisch einerseits leicht verarbeitbar und andererseits haut- und körperneutral bzw. -pflegend ist. Kosmetische Zubereitungen unter Verwendung des erfindungsgemäßen Polyglycerinfettsäureestergemisches vermitteln ein optimales pflegendes Gefühl. Bei der Anwendung auf der Haut, z.B. in kosmetischen Zubereitungen, Hautdesinfektionsmitteln, Salben, Einreibungen und dergleichen hat das erfindungsgemäße Polyglycerinfettsäureestergemisch eine rückfettende Wirkung.

Des weiteren eignet sich das erfindungsgemäße Polyglycerinfettsäureestergemisch vorzüglich für technische Anwendungen, vorzugsweise als Emulgator in Bohrölen oder Bohrflüssigkeiten sowie Schmierölen, sowie als Netz- und/oder Dispergiermittel in technischen Reinigungsmitteln oder als Emulgator und/oder Dispergiermittel in Farbzubereitungen, vorzugsweise in Dispersionsfarben, oder Bautenschutzmitteln, insbesondere Holzschutzfarben und -lasuren.

Außerdem kann das erfindungsgemäße Polyglycerinfettsäureestergemisch als wasserlösliches Schmiermittel in chemisch-technischen Zubereitungen eingesetzt werden.

In anwendungsfertigen Formulierungen kann das erfindungsgemäße Polyglycerinfettsäureestergemisch mit weiteren Zusatz- oder Hilfsmitteln problemlos kombiniert werden. In Wasch-, Reinigungs- und/oder Körperreinigungsmittel können bevorzugt zusätzlich Elektrolyte und/oder weitere Tenside sowie Lösungs- und/oder Verdünnungsmittel eingesetzt werden. Des weiteren kann das erfindungsgemäße Polyglycerinfettsäureestergemisch zusammen mit Konservierungs-, Parfümierungs-, Färbemitteln, pharmazeutischen Wirkstoffen, Stellmitteln zur pH-Regulierung, Komplexierungsmitteln zur Maskierung von Metallionen, Hautpflegemitteln und/oder Verdickungsmitteln und anderen Verbindungen, wie Kolloiden, desinfizierenden Wirkstoffen, fungiziden, insektiziden oder antibakteriellen Wirkstoffen, Korrosionsschutzmitteln usw., verwendet werden.

Die nachfolgenden Ausführungs- und Anwendungsbeispiele sollen die Erfindung erläutern, ohne sie darauf zu beschränken.

### 1. Ausführungsbeispiel

### Herstellung von Polyglycerincaprinat

In einem 4-Liter-Vierhalskolben, der mit Rührwerk, Wasserabscheider, Thermometer und Gaseinleitungsrohr versehen ist, wird eine Mischung von 2608 g Polyglycerin (mittleres Molekulargewicht: ca. 326 g/mol; bestehend aus ca. 30 % Triglycerin, ca. 45 % Tetraglycerin, ca. 17 % Pentaglycerin, und ca. 8 % höheren Oligomeren), 688 g = 4 mol Caprinsäure, 5,6 g Dodecylbenzolsulfonsäure und 2,2 g hypophosphorige Säure unter Rühren und gleichzeitigem Durchleiten von Inertgas während ca. 3 h bei 400 bis 40 mbar bis max. 155°C erhitzt, wobei das Reaktionswasser kontinuierlich destillativ entfernt wird. Bei Erreichen einer Säurezahl von < 2 wird das Reaktionsgemisch auf ca. 65°C heruntergekühlt und nicht umgesetzte Anteile an Polyglycerin werden wie folgt entfernt:

Das Reaktionsgemisch wird in Ethylacetat (halber Volumenanteil) gelöst und mit Wasser extrahiert. Dem Wasser wird die der Säurezahl entsprechende Menge an Natronlauge zugesetzt. Die Menge des zur Extraktion verwendeten Wassers beträgt ca. 50 Vol.-% des eingesetzten Esters. Die Summe der extrahierten Anteile an Polyglycerin nach Abdestillation der Lösungsmittel beträgt ca. 20 Gew.-% des eingesetzten Rohesters. Die nach der Extraktion verbleibende organische Phase wird im Vakuum eingedampft (Restgehalt an Wasser: 10 Gew.-%).

### Kennzahlen

| | |
|---|---|
| Säurezahl: | < 2 |
| Verseifungszahl: | 75 - 85 |
| HLB-Wert: | 14,3 |

### 2 . Ausführungsbeispiel

### Herstellung von Polyglycerinlaurat

In einem 2-Liter-Vierhalskolben, der mit Rührwerk, Wasserabscheider, Thermometer und Gaseinleitungsrohr versehen ist, wird eine Mischung von 886 g Polyglycerin (mittleres Molekulargewicht: ca. 326 g/mol; bestehend aus ca. 30 % Triglycerin, ca. 45 % Tetraglycerin, ca. 17 % Pentaglycerin und ca. 8 % höheren Oligomeren), 272 g = 1,36 mol Laurinsäure, 2,8 g Dodecylbenzolsulfonsäure und 1,1 g hypophosphorige Säure unter Rühren und gleichzeitigem Durchleiten von Inertgas während ca. 3 h bei 400 bis 40 mbar bis max. 155°C erhitzt, wobei das Reaktionswasser kontinuierlich destillativ entfernt wird. Bei Erreichen einer Säurezahl von < 2 wird das Reaktionsgemisch auf ca. 70°C heruntergekühlt und nicht umgesetzte Anteile an Polyglycerin werden wie folgt entfernt:

Das Reaktionsgemisch wird in Ethylacetat (halber Volumenanteil) gelöst und mit Wasser extrahiert. Dem Wasser wird die der Säurezahl entsprechende Menge an Natronlauge zugesetzt. Die Menge des zur Extraktion verwendeten Wassers beträgt ca. 50 Vol.-% des eingesetzten Esters. Die Summe der extrahierten Anteile an Polyglycerin nach Abdestillation der Lösungsmittel beträgt ca. 31 Gew.-% des eingesetzten Rohesters. Die nach der Extraktion verbleibende organische Phase wird im Vakuum eingedampft.

### Kennzahlen

| | |
|---|---|
| Säurezahl: | 0,9 |
| Verseifungszahl: | 98,4 |
| HLB-Wert: | 13,0 |

### 3. Ausführungsbeispiel

### Herstellung von Polyglycerincaprylcaprinat

In einem 4-Liter-Vierhalskolben, der mit Rührwerk, Wasserabscheider, Thermometer und Gaseinleitungsrohr versehen ist, wird eine Mischung von 2457 g Polyglycerin (mittleres Molekulargewicht: ca. 273 g/mol; bestehend aus ca. 4 % Diglycerin, ca. 62 % Triglycerin, ca. 24 % Tetraglycerin, ca. 7 % Pentaglycerin und ca. 3 % höheren Oligomeren), 706 g = 4,5 mol Capryl-/Caprinsäuregemisch, 6,3 g Dodecylbenzolsulfonsäure und 2,5 g hypophosphorige Säure unter Rühren und gleichzeitigem Durchleiten von Inertgas während ca. 3 h bei 400 bis 40 mbar bis max. 155°C erhitzt, wobei das Reaktionswasser kontinuierlich destillativ entfernt wird. Bei Erreichen einer Säurezahl von < 2 wird das Reaktionsgemisch auf ca. 70°C heruntergekühlt und nicht umgesetzte Anteile an Polyglycerin werden wie folgt entfernt:

Das Reaktionsgemisch wird in Ethylacetat (halber Volumenanteil) gelöst und mit Wasser extrahiert. Dem Wasser wird die der Säurezahl entsprechende Menge an Natronlauge zugesetzt. Die Menge des zur Extraktion verwendeten Wassers beträgt ca. 50 Vol.-% des eingesetzten Esters. Die Summe der extrahierten Anteile an Polyglycerin nach Abdestillation der Lösungsmittel beträgt ca. 25 Gew.-% des eingesetzten Rohesters. Die nach der Extraktion verbleibende organische Phase wird im Vakuum eingedampft.

### Kennzahlen

| | |
|---|---|
| Säurezahl: | 1,0 |
| Verseifungszahl: | 108,9 |
| HLB-Wert: | 12,3 |

### Anwendungsbeispiele für das erfindungsgemäße Polyglycerinfettsäureestergemisch

### 1. O/W-Körperlotion mit dem erfindungsgemäßen Polyglycerinlaurat als O/W-Emulgator

- 1,5 Gew.-%: erf.-gem. Polyglycerinlaurat (hergestellt gemäß Ausführungsbeispiel 2)
- 0,5 Gew.-%: Natriumpolyacrylat
- 5,0 Gew.-%: Paraffinol dickfl. DAB
- 4,0 Gew.-%: acetyliertes Lanolin
- 6,0 Gew.-%: Cetyl-Stearylisononanoat
- 4,0 Gew.-%: Diglycerin
- 0,05 Gew.-%: Konservierungsmittel
- 0,3 Gew.-%: Parfüm
- 78,65 Gew.-%: Wasser (vollentsalzt)

Das Produkt ist eine weiße, stabile Emulsion mit sehr gutem Einziehvermögen und hervorragendem Hautgefühl.

Zentrifugentest 20 Min. 5000 UpM: Keine Abtrennung Viskosität, gemessen mit Haake-Viskosimeter, VT 181, MV DIN/32 bei 20°C: 3500 mPa.s

### 2. O/W-Körperlotion mit natürlichen bzw. naturidentischen Rohstoffen und dem erfindungsgemäßen Polyglycerinlaurat als O/W-Emulgator

- 2,0 Gew.-%: erf.-gem. Polyglycerinlaurat (hergestellt gemäß Ausführungsbeispiel 2)
- 0,5 Gew.-%: Natriumpolyacrylat 6,0 Gew.-%
- 6,0 Gew.-%: Weizenkeimöl
- 6,0 Gew.-%: Cetyl-Stearylisooctanoat (Bürzeldrüsenöl synthetisch)
- 7,0 Gew.-%: Capryl-Caprinsäuretriglycerid
- 4,0 Gew.-%: Diglycerin
- 0,05 Gew.-%: Konservierungsmittel
- 0,3 Gew.-%: Parfüm
- 74,15 Gew.-%: Wasser (vollentsalzt)

Das Produkt ist eine schwachgelbe, glänzende, stabile Emulsion.

Zentrifugentest 20 Min. 5000 UpM: Keine Abtrennung Viskosität gemessen mit Haake-Viskosimeter VT 181, MV DIN/32 bei 20°C: 3000 mPa.s

### 3. Rosmarinbadeöl

- 27 Gew.-%: Rosmarinöl
- 33 Gew.-%: erf.-gem. Polyglycerincaprinat (hergestellt gemäß Ausführungsbeispiel 1)
- 40 Gew.-%: Wasser (vollentsalzt)

Das Produkt ist ein blankes, niedrigviskoses Öl mit gutem Emulgiervermögen in warmem Wasser und gutem Rückfettungsvermögen auf der Haut.

### 4. Fichtennadelbadeöl

- 20 Gew.-%: Fichtennadelöl
- 45 Gew.-%: erf.-gem. Polyglycerincaprylcaprinat (hergestellt gemäß Ausführungsbeispiel 3)
- 35 Gew.-%: Wasser (vollentsalzt)

Das Produkt ist ein blankes, niedrig viskoses Öl mit gutem Emulgiervermögen in warmem Wasser und gutem Rückfettungsvermögen auf der Haut.

### 5. Rosmarinbadeöl mit Wasseranteil

- 20 Gew.-%: Rosmarinöl
- 50 Gew.-%: erf.-gem. Polyglycerincaprinat (hergestellt gemäß Ausführungsbeispiel 1)
- 30 Gew.-%: Wasser (vollentsalzt)

Das Produkt ist ein blankes, niedrigviskoses Öl mit gutem Emulgiervermögen in warmem Wasser und gutem Rückfettungsvermögen auf der Haut.

### 6. Ölbadpräparat mit hoher Pflegewirkung, spreitend

- 20 Gew.-%: erf.-gem. Polyglycerincaprinat (hergestellt gemäß Ausführungsbeispiel 1)
- 20 Gew.-%: Polyglycerincaprinat (hergestellt gemäß P 41 05 305.2)
- 20 Gew.-%: Isopropylmyristat
- 31 Gew.-%: Paraffinöl DAB dickfl.
- 5 Gew.-%: Parfümöl
- 4 Gew.-%: Wasser (vollentsalzt)

### 7. Pflegendes Badeöl, spontanemulgierend

- 25 Gew.-%: erf.-gem. Polyglycerincaprinat (hergestellt gemäß Ausführungsbeispiel 1)
- 17 Gew.-%: Polyglycerincocoat (hergestellt gemäß P 41 05 305.2)
- 33 Gew.-%: Isopropylmyristat
- 20 Gew.-%: Jojobaöl
- 5 Gew.-%: Parfümöl

## Patentansprüche

1. Polyglycerinfettsäureestergemisch aus Mono-, Di- und Polyestern von Polyglycerinen mit einem Polymerisationsgrad von 2 bis 8 und mindestens einer gesättigten und/oder ungesättigten Fettsäure, dadurch gekennzeichnet, daß das Polyglycerinfettsäureestergemisch (bezogen auf 100 Gewichtsteile des Polyglycerinfettsäureestergemisches) einen Anteil von
20 bis 65 Gew.-% Triglycerinfettsäureestern,
20 bis 50 Gew.-% Tetraglycerinfettsäureestern und
5 bis 40 Gew.-% höheren Polyglycerinfettsäureestern
aufweist und keine oder nur geringe Mengen (weniger als 5 Gew.-%) an Diglycerinfettsäureestern enthält, wobei
die Fettsäurekomponente aus einer oder mehreren Fettsäuren, ausgewählt aus den gesättigten und/oder ungesättigten, verzweigten und/oder unverzweigten C₆-bis C₁₄-Fettsäuren, mit einem Anteil unter 10 Gew.-% an Fettsäuren mit mehr als 14 C-Atomen, besteht,
der HLB-Wert des Polyglycerinfettsäureestergemisches größer als 8 ist.

2. Polyglycerinfettsäureestergemisch nach Anspruch 1, dadurch gekennzeichnet, daß das Polyglycerinfettsäureestergemisch (bezogen auf 100 Gewichtsteile des Polyglycerinfettsäureestergemisches) einen Anteil von
22 bis 32 Gew.-% Triglycerinfettsäureestern,
39 bis 49 Gew.-% Tetraglycerinfettsäureestern und
24 bis 34 Gew.-% höheren Polyglycerinfettsäureestern
aufweist und keine oder nur geringe Mengen (weniger als 3 Gew.-%) an Diglycerinfettsäureestern enthält, wobei
die Fettsäurekomponente aus einer oder mehreren Fettsäuren, ausgewählt aus den gesättigten und/oder ungesättigten, verzweigten und/oder unverzweigten C₈-bis C₁₂-Fettsäuren, mit einem Anteil unter 5 Gew.-% an Fettsäuren mit mehr als 14 C-Atomen, besteht,
der HLB-Wert des Polyglycerinfettsäureestergemisches größer als 10 ist.

3. Polyglycerinfettsäureestergemisch nach den Ansprüchen 1 oder 2, dadurch gekennzeichnet, daß es einen Anteil 5 bis 20 Gew.-% Pentaglycerinfettsäureestern aufweist.

4. Polyglycerinfettsäureestergemisch nach einem oder mehreren der Ansprüche 1 bis 3, dadurch gekennzeichnet, daß die Fettsäurekomponente aus Caprylsäure, Caprinsäure, Laurinsäure, Undecensäure, 2-Ethylhexansäure und/oder Kokosfettsäure besteht.

5. Polyglycerinfettsäureestergemisch nach einem oder mehreren der Ansprüche 1 bis 4, dadurch gekennzeichnet, daß es zu mehr als 50 Gew.-% aus Mono- und Diestern besteht.

6. Badezusatzpräparat, insbesondere Ölbadpräparat, enthaltend
ein Polyglycerinfettsäureestergemisch aus Mono-, Di-und Polyestern von Polyglycerinen mit einem Polymerisationsgrad von 2 bis 8 und mindestens einer gesättigten und/oder ungesättigten Fettsäure,
mindestens eine ölige oder ölhaltige Komponente, ausgewählt aus den naturlichen und synthetischen, mineralischen, ätherischen und fetten tierischen und pflanzlichen ölen,
gegebenenfalls ein Lösemittel oder Lösemittelgemisch sowie gegebenenfalls weitere Hilfs- und Zusatzstoffe, dadurch gekennzeichnet, daß es
10 bis 60 Gew.-% eines Polyglycerinfettsäureestergemisches als wasserlöslichen Emulgator und/oder Solubilisator,
10 bis 60 Gew.-% eines kosmetisch und/oder therapeutisch wirkenden Öls, Ölgemisches und/oder ölkomponente und
0 bis 70 Gew.-% Wasser
enthält, wobei das Polyglycerinfettsäureestergemisch (bezogen auf 100 Gewichtsteile des Polyglycerinfettsäureestergemisches) einen Anteil von
20 bis 65 Gew.-% Triglycerinfettsäureestern,
20 bis 50 Gew.-% Tetraglycerinfettsäureestern und
5 bis 40 Gew.-% höheren Polyglycerinfettsäureestern
aufweist und keine oder nur geringe Mengen (weniger als 5 Gew.-%) an Diglycerinfettsäureestern enthält und die Fettsäurekomponente aus einer oder mehreren Fettsäuren, ausgewählt aus den gesättigten und/oder ungesättigten, verzweigten und/oder unverzweigten C₆- bis C₁₄-Fettsäuren, mit einem Anteil unter 10 Gew.-% an Fettsäuren mit mehr als 14 C-Atomen, besteht.

7. Badezusatzpräparat nach Anspruch 6, dadurch gekennzeichnet, daß es
15 bis 50 Gew.-% eines Polyglycerinfettsäureestergemisches,
15 bis 50 Gew.-% eines kosmetisch und/oder therapeutisch wirkenden Öls, ölgemisches und/oder ölkomponente und
0,5 bis 60 Gew.-% Wasser
enthält, wobei das Polyglycerinfettsäureestergemisch (bezogen auf 100 Gewichtsteile des Polyglycerinfettsäureestergemisches) einen Anteil von
22 bis 32 Gew.-% Triglycerinfettsäureestern,
39 bis 49 Gew.-% Tetraglycerinfettsäureestern und
24 bis 34 Gew.-% höheren Polyglycerinfettsäureestern
aufweist und keine oder nur geringe Mengen (weniger als 3 Gew.-%) an Diglycerinfettsäureestern enthält und die Fettsäurekomponente aus einer oder mehreren Fettsäuren, ausgewählt aus den gesättigten und/oder ungesättigten, verzweigten und/oder unverzweigten C₈- bis C₁₂-Fettsäuren, mit einem Anteil unter 5 Gew.-% an Fettsäuren mit mehr als 14 C-Atomen, besteht.

8. Verfahren zur Herstellung eines Polyglycerinfettsäureestergemisches aus Mono-, Di- und Polyestern von Polyglycerinen mit einem Polymerisationsgrad von 2 bis 8 und mindestens einer gesättigten und/oder ungesättigten Fettsäure, dadurch gekennzeichnet, daß ein Polyglycerin, das (bezogen auf 100 Gew.-Teile Polyglycerin) einen Anteil von
20 bis 65 Gew.-% Triglycerin,
20 bis 50 Gew.-% Tetraglycerin und
5 bis 40 Gew.-% höheren Polyglycerinen
aufweist und keine oder nur geringe Mengen (weniger als 5 Gew.-%) an Diglycerin enthält, mit einer oder mehreren Fettsäuren, ausgewählt aus den gesättigten und/oder ungesättigten, verzeigten und/oder unverzeigten C₆- bis C₁₄-Fettsäuren, wobei die Fettsäure oder das Fettsäuregemisch einen Anteil von unter 10 Gew.-% an Fettsäuren mit mehr als 14 C-Atomen aufweist, in einem Molverhältnis des Polyglycerins zu der Fettsäure oder dem Fettsäuregemisch von
4 : 1 bis 1 : 1, vorzugsweise
2,5 : 1 bis 1,5 : 1
in Gegenwart mindestens eines Katalysators, vorzugsweise eines sauren Katalysators, und bei Unterdruck umgesetzt wird, indem das Reaktionsgemisch zunächst bis auf 140°C, vorzugsweise 145°C, aufgeheizt und der Druck bis auf 600 mbar, vorzugsweise 500 mbar, reduziert wird und dann das Reaktionsgemisch in einem Temperaturbereich von
140 bis 220°C, vorzugsweise
145 bis 190°C,
stufenweise oder kontinuierlich, über ein Temperaturprogramm gesteuert, erhitzt und dabei der Druck stufenweise oder kontinuierlich, über ein Druckprogramm gesteuert,
von 600 auf 10 mbar, vorzugsweise
von 500 auf 20 mbar,
erniedrigt wird, wobei das entstehende Reaktionswasser kontinuierlich abdestilliert und bei Erreichen einer Säurezahl von < 3 das erhaltene Polyglycerinfettsäureestergemisch abgekühlt und gegebenenfalls aufgearbeitet und/oder gereinigt wird.

9. Verfahren nach Anspruch 8, dadurch gekennzeichnet, daß das eingesetzte Polyglycerin (bezogen auf 100 Gew.-teile Polyglycerin) einen Anteil von
22 bis 32 Gew.-% Triglycerin,
39 bis 49 Gew.-% Tetraglycerin und
24 bis 34 Gew.-% höheren Polyglycerinen
aufweist und keine oder nur geringe Mengen (weniger als 3 Gew.-%) an Diglycerin enthält.

10. Verfahren nach den Ansprüchen 8 oder 9, dadurch gekennzeichnet, daß eine Fettsäure oder ein Fettsäuregemisch, ausgewählt aus den gesättigten und/oder ungesättigten, verzeigten und/oder unverzeigten C₈- bis C₁₂-Fettsäuren, mit einem Anteil unter 5 Gew-% an Fettsäuren mit mehr als 14 C-Atomen, eingesetzt wird.

11. Verfahren nach einem oder mehreren der Ansprüche 8 bis 10, dadurch gekennzeichnet, daß das erhaltene Polyglycerinfettsäureestergemisch auf 30 bis 110°C, vorzugsweise 60 bis 80°C, abgekühlt und nachfolgend teilweise von nicht umgesetzten Polyglycerinanteilen durch Extraktion befreit wird, indem das Polyglycerinfettsäureestergemisch mit einem organisch-chemischen Lösungsmittel oder Lösemittelgemisch versetzt und anschliessend mit Wasser in vorzugsweise einer Extraktionsstufe Polyglycerin extrahiert wird, wobei in der ersten Extraktionsstufe zusätzlich eine der Säurezahl des Polyglycerinfettsäureestergemisches entsprechende und/oder der Katalysatormenge mindestens äquivalente Gewichtsmenge einer anorganischen und/oder organischen, basisch reagierenden Verbindung zugesetzt wird, und die nach der Extraktion verbleibende organische Phase von dem eingesetzten organischen Lösungsmittel und dem Restwassergehalt durch Destillation, vorzugsweise Vakuumdestillation oder Vakuumverdampfung, befreit wird.

12. Verfahren nach einem oder mehreren der Ansprüche 8 bis 11, dadurch gekennzeichnet, daß das organisch-chemische Lösemittel oder Lösemittelgemisch ein Wasseraufnahmevermögen von weniger als 30 Gew.-%, vorzugsweise weniger als 20 Gew.-% (bezogen auf 100 Gew.-Teile des organisch-chemischen Lösemittels oder Lösemittelgemisches), aufweist und/oder mit Wasser bei der Destillation oder in der Gasphase ein azeotropes Gemisch bildet.

13. Verfahren nach einem oder mehreren der Ansprüche 8 bis 12, dadurch gekennzeichnet, daß als organisch-chemisches Lösemittel Ethylacetat, vorzugsweise mit Wasser gesättigtes Ethylacetat, eingesetzt wird.

14. Verfahren nach einem oder mehreren der Ansprüche 8 bis 13, dadurch gekennzeichnet, daß zur Neutralisation des Katalysators Natriumhydroxid, vorzugsweise eine wäßrige Natriumhydroxidlösung, in der ersten Extraktionsstufe dem organisch-chemischen Lösemittel oder Lösemittelgemisch und/oder der Wasserphase zugegeben wird.

15. Verfahren nach einem oder mehreren der Ansprüche 8 bis 14, dadurch gekennzeichnet, daß zur Neutralisation des Katalysators Alkalicarbonate, vorzugsweise Natriumcarbonat und/oder Kaliumcarbonat, und/oder ein basischer Ionenaustauscher, in der ersten Extraktionsstufe dem organisch-chemischen Lösemittel oder Lösemittelgemisch und/oder der Wasserphase zugegeben werden.

16. Verfahren nach einem oder mehreren der Ansprüche 8 bis 15, dadurch gekennzeichnet, daß das erhaltene Polyglycerinfettsäureestergemisch vor einer Weiterverarbeitung und/oder Reinigung mehr als 0,5 h, vorzugsweise 1 bis 10 h, stehengelassen wird und gegebenenfalls ausgeschiedene Anteile an Polyglycerinen abgetrennt werden.

17. Verfahren nach einem oder mehreren der Ansprüche 8 bis 16, dadurch gekennzeichnet, daß die Aufheizung des Reaktionsgemisches im Temperaturbereich von 140 bis 220°c, vorzugsweise 145 bis 190°C, und die Druckerniedrigung von 600 auf 10 mbar, vorzugsweise von 500 auf 20 mbar, in einem Zeitintervall von
2 bis 6 Stunden, vorzugsweise
3 bis 4 Stunden,
erfolgen.

18. Verfahren nach einem oder mehreren der Ansprüche 8 bis 17, dadurch gekennzeichnet, daß die Aufheizung des Reaktionsgemisches im Temperaturbereich von 140 bis 220°C, vorzugsweise 145 bis 190°C, stufenweise über
3 bis 6 Stufen, vorzugsweise
4 bis 5 Stufen,
erfolgt.

19. Verfahren nach einem oder mehreren der Ansprüche 8 bis 18, dadurch gekennzeichnet, daß die Druckerniedrigung von 600 auf 10 mbar, vorzugsweise von 500 auf 20 mbar, stufenweise über
3 bis 6 Stufen, vorzugsweise
4 bis 5 Stufen,
erfolgt.

20. Verfahren nach einem oder mehreren der Ansprüche 8 bis 19, dadurch gekennzeichnet, daß der saure Katalysator, vorzugsweise eine sulfonsäuregruppenhaltige Verbindung, in Kombination mit einer sauren, reduzierend wirkenden Verbindung, vorzugsweise hypophosphoriger Säure, eingesetzt wird.

21. Verfahren nach einem oder mehreren der Ansprüche 8 bis 20, dadurch gekennzeichnet, daß das Reaktionsgemisch in Gegenwart eines inerten Gases, vorzugsweise Stickstoff, umgesetzt wird.

22. Verwendung des Polyglycerinfettsäureestergemisches nach einem oder mehreren der Ansprüche 1 bis 5 als Emulgator, Solubilisierungs-, Dispergierungs-, Netzmittel und/oder Rückfetter in kosmetischen, pharmazeutischen oder chemisch-technischen Zubereitungen.

23. Verwendung des Polyglycerinfettsäureestergemisches nach Anspruch 22 zur Solubilisierung und/oder Emulgierung von natürlichen oder synthetischen, ätherischen, mineralischen, fetten tierischen oder pflanzlichen Ölen.

24. Verwendung des Polyglycerinfettsäureestergemisches nach Anspruch 22 als Emulgator in Bohrölen oder Bohrflüssigkeiten.

25. Verwendung des Polyglycerinfettsäureestergemisches nach Anspruch 22 als Netz- und/oder Dispergiermittel in technischen Reinigungsmitteln.

26. Verwendung des Polyglycerinfettsäureestergemisches nach Anspruch 22 als Emulgator und/oder Dispergiermittel in Farbzubereitungen, vorzugsweise in Dispersionsfarben.

27. Verwendung des Polyglycerinfettsäureestergemisches nach einem oder mehreren der Ansprüche 1 bis 5 als Hautpflegeadditiv und/oder Wasch-, Reinigungs- oder Körperreinigungsmittel.

28. Verwendung des Polyglycerinfettsäureestergemisches nach einem oder mehreren der Ansprüche 1 bis 5 als wasserlösliches Schmiermittel im chemisch-technischen Zubereitungen.

## Claims

1. A polyglycerol fatty acid ester mixture consisting of monoesters, diesters and polyesters of polyglycerols having a degree of polymerisation of 2 to 8 and at least one saturated and/or unsaturated fatty acid, characterised in that the polyglycerol fatty acid ester mixture (relative to 100 parts by weight of the polyglycerol fatty acid ester mixture) has a content of
20 to 65% by weight triglycerol fatty acid esters,
20 to 50% by weight tetraglycerol fatty acid esters, and
5 to 40% by weight higher polyglycerol fatty acid esters
and contains no diglycerol fatty acid esters or only small quantities (less than 5% by weight) of diglycerol fatty acid esters,
wherein
the fatty acid component consists of one or more fatty acids, selected from the saturated and/or unsaturated, branched and/or unbranched C₆ to C₁₄ fatty acids, with a content of less than 10% by weight of fatty acids having more than 14 C atoms,
the HLB value of the polyglycerol fatty acid ester mixture is greater than 8.

2. A polyglycerol fatty acid ester mixture according to Claim 1, characterised in that the polyglycerol fatty acid ester mixture (relative to 100 parts by weight of the polyglycerol fatty acid ester mixture) has a content of
22 to 32% by weight triglycerol fatty acid esters,
39 to 49% by weight tetraglycerol fatty acid esters, and
24 to 34% by weight higher polyglycerol fatty acid esters
and contains no diglycerol fatty acid esters or only small quantities (less than 3% by weight) of diglycerol fatty acid esters,
wherein
the fatty acid component consists of one or more fatty acids, selected from the saturated and/or unsaturated, branched and/or unbranched C₈ to C₁₂ fatty acids, with a content of less than 5% by weight of fatty acids having more than 14 C atoms,
the HLB value of the polyglycerol fatty acid ester mixture is greater than 10.

3. A polyglycerol fatty acid ester mixture according to Claims 1 or 2, characterised in that it has a content of 5 to 20% by weight pentaglycerol fatty acid esters.

4. A polyglycerol fatty acid ester mixture according to one or more of Claims 1 to 3, characterised in that the fatty acid component consists of caprylic acid, capric acid, lauric acid, undecenoic acid, 2-ethylhexanoic acid and/or coco fatty acid.

5. A polyglycerol fatty acid ester mixture according to one or more of Claims 1 to 4, characterised in that it consists of more than 50% by weight monoesters and diesters.

6. A bath additive preparation, in particular oil bath preparation, containing
a polyglycerol fatty acid ester mixture consisting of monoesters, diesters and polyesters of polyglycerols having a degree of polymerisation of 2 to 8 and at least one saturated and/or unsaturated fatty acid,
at least one oily or oil-containing component, selected from among the natural and synthetic, mineral, ethereal and fatty animal and vegetable oils,
optionally a solvent or solvent mixture and also optionally other auxiliaries and additives, characterised in that it contains
10 to 60% by weight of a polyglycerol fatty acid ester mixture as water-soluble emulsifier and/or solubiliser,
10 to 60% by weight of a cosmetic and/or therapeutic oil, oil mixture and/or oil component, and
0 to 70% by weight water,
wherein the polyglycerol fatty acid ester mixture (relative to 100 parts by weight of the polyglycerol fatty acid ester mixture) has a content of
20 to 65% by weight triglycerol fatty acid esters,
20 to 50% by weight tetraglycerol fatty acid esters, and
5 to 40% by weight higher polyglycerol fatty acid esters,
and contains no diglycerol fatty acid esters or only small quantities (less than 5% by weight) of diglycerol fatty acid esters, and the fatty acid component consists of one or more fatty acids, selected from the saturated and/or unsaturated, branched and/or unbranched C₆ to C₁₄ fatty acids, with a content of less than 10% by weight of fatty acids having more than 14 C atoms.

7. A bath additive preparation according to Claim 6, characterised in that it contains
15 to 50% by weight of a polyglycerol fatty acid ester mixture,
15 to 50% by weight of a cosmetic and/or therapeutic oil, oil mixture and/or oil component, and
0.5 to 60% by weight water,
wherein the polyglycerol fatty acid ester mixture (relative to 100 parts by weight of the polyglycerol fatty acid ester mixture) has a content of
22 to 32% by weight triglycerol fatty acid esters,
39 to 49% by weight tetraglycerol fatty acid esters, and
24 to 34% by weight higher polyglycerol fatty acid esters,
and contains no diglycerol fatty acid esters or only small quantities (less than 3% by weight) of diglycerol fatty acid esters, and the fatty acid component consists of one or more fatty acids, selected from the saturated and/or unsaturated, branched and/or unbranched C₈ to C₁₂ fatty acids, with a content of less than 5% by weight of fatty acids having more than 14 C atoms.

8. A method for preparing a polyglycerol fatty acid ester mixture consisting of monoesters, diesters and polyesters of polyglycerols having a degree of polymerisation of 2 to 8 and at least one saturated and/or unsaturated fatty acid, characterised in that a polyglycerol which (relative to 100 parts by weight polyglycerol) has a content of
20 to 65% by weight triglycerol,
20 to 50% by weight tetraglycerol, and
5 to 40% by weight higher polyglycerols
and contains no diglycerol or only small quantities (less than 5% by weight) of diglycerol, is reacted with one or more fatty acids, selected from the saturated and/or unsaturated, branched and/or unbranched C₆ to C₁₄ fatty acids, the fatty acid or the fatty acid mixture having a content of less than 10% by weight of fatty acids having more than 14 C atoms, in a molar ratio of the polyglycerol to the fatty acid or the fatty acid mixture of
4 : 1 to 1 : 1, preferably
2.5 : 1 to 1.5 : 1,
in the presence of at least one catalyst, preferably an acidic catalyst, and at reduced pressure, by first heating the reaction mixture to 140°C, preferably 145°C, and reducing the pressure to 600 mbar, preferably 500 mbar, and then the reaction mixture is heated in a temperature range of
140 to 220°C, preferably
145 to 190°C,
in steps or continuously, controlled by a temperature program, and during this the pressure is reduced in steps or continuously, controlled by a pressure program,
from 600 to 10 mbar, preferably
from 500 to 20 mbar,
the resulting reaction water being continuously distilled off and when an acid number of < 3 is reached the resulting polyglycerol fatty acid ester mixture is cooled and optionally worked up and/or purified.

9. A method according to Claim 8, characterised in that the polyglycerol used (relative to 100 parts by weight polyglycerol) has a content of
22 to 32% by weight triglycerol,
39 to 49% by weight tetraglycerol and
24 to 34% by weight higher polyglycerols
and contains no diglycerol or only small quantities (less than 3% by weight) of diglycerol.

10. A method according to Claims 8 or 9, characterised in that a fatty acid or a fatty acid mixture, selected from the saturated and/or unsaturated, branched and/or unbranched C₈ to C₁₂ fatty acids, having a content of less than 5% by weight fatty acids with more than 14 C atoms, is used.

11. A method according to one or more of Claims 8 to 10, characterised in that the resulting polyglycerol fatty acid ester mixture is cooled to 30 to 110°C, preferably 60 to 80°C, and is subsequently partly freed of non-reacted polyglycerol contents by extraction, in that an organic-chemical solvent or solvent mixture is added to the polyglycerol fatty acid ester mixture and then polyglycerol is extracted with water in preferably one extraction stage, with a quantity by weight of an inorganic and/or organic, basic-reacting compound which corresponds to the acid number of the polyglycerol fatty acid ester mixture and/or is at least equivalent to the quantity of catalyst additionally being added in the first extraction stage, and the organic phase remaining after extraction being freed from the organic solvent used and the residual water content by distillation, preferably vacuum distillation or vacuum evaporation.

12. A method according to one or more of Claims 8 to 11, characterised in that the organic-chemical solvent or solvent mixture has a water absorption capacity of less than 30% by weight, preferably less than 20% by weight (relative to 100 parts by weight of the organic-chemical solvent or solvent mixture) and/or forms an azeotropic mixture with water upon distillation or in the gas phase.

13. A method according to one or more of Claims 8 to 12, characterised in that ethyl acetate, preferably ethyl acetate saturated with water, is used as the organic-chemical solvent.

14. A method according to one or more of Claims 8 to 13, characterised in that for neutralisation of the catalyst sodium hydroxide, preferably an aqueous sodium hydroxide solution, is added in the first extraction stage to the organic-chemical solvent or solvent mixture and/or the aqueous phase.

15. A method according to one or more of Claims 8 to 14, characterised in that for neutralisation of the catalyst alkali carbonates, preferably sodium carbonate and/or potassium carbonate, and/or a basic ion exchanger, are added in the first extraction stage to the organic-chemical solvent or solvent mixture and/or the aqueous phase.

16. A method according to one or more of Claims 8 to 15, characterised in that the resulting polyglycerol fatty acid ester mixture is allowed to stand for more than 0.5 hours, preferably 1 to 10 hours, before further processing and/or purification, and optionally contents of polyglycerols which have separated out are separated off.

17. A method according to one or more of Claims 8 to 16, characterised in that the heating of the reaction mixture in a temperature range of 140 to 220°C, preferably 145 to 190°C, and the reduction of pressure from 600 to 10 mbar, preferably from 500 to 20 mbar, is effected in a time interval of
2 to 6 hours, preferably
3 to 4 hours.

18. A method according to one or more of Claims 8 to 17, characterised in that the heating of the reaction mixture in the temperature range of 140 to 220°C, preferably 145 to 190°C, is effected in stages over
3 to 6 stages, preferably
4 to 5 stages.

19. A method according to one or more of Claims 8 to 18, characterised in that the reduction of pressure from 600 to 10 mbar, preferably from 500 to 20 mbar, is effected in stages over
3 to 6 stages, preferably
4 to 5 stages.

20. A method according to one or more of Claims 8 to 19, characterised in that the acidic catalyst, preferably a compound containing sulphonic acid groups, is used in combination with an acidic reducing compound, preferably hypophosphorous acid.

21. A method according to one or more of Claims 8 to 20, characterised in that the reaction mixture is reacted in the presence of an inert gas, preferably nitrogen.

22. The use of the polyglycerol fatty acid ester mixture according to one or more of Claims 1 to 5 as an emulsifier, solubiliser, dispersing agent, wetting agent and/or regreasing agent in cosmetic, pharmaceutical or chemical-technological preparations.

23. The use of the polyglycerol fatty acid ester mixture according to Claim 22 for the solubilisation and/or emulsification of natural or synthetic, ethereal, mineral, fatty animal or vegetable oils.

24. The use of the polyglycerol fatty acid ester mixture according to Claim 22 as an emulsifier in drilling oils or drilling fluids.

25. The use of the polyglycerol fatty acid ester mixture according to Claim 22 as a wetting and/or dispersing agent in industrial cleaning agents.

26. The use of the polyglycerol fatty acid ester mixture according to Claim 22 as an emulsifier and/or dispersing agent in paint preparations, preferably in emulsion paints.

27. The use of the polyglycerol fatty acid ester mixture according to one or more of Claims 1 to 5 as a skin-care additive and/or washing, cleansing or body-cleansing agent.

28. The use of the polyglycerol fatty acid ester mixture according to one or more of Claims 1 to 5 as a water-soluble lubricant in chemical-technological preparations.

## Revendications

1. Mélange d'esters d'acides gras des polyglycérols, constitué de mono-, di- et polyesters de polyglycérols, avec un degré de polymérisation de 2 à 8, et d'au moins un acide gras saturé et/ou insaturé, caractérisé en ce que le mélange d'esters d'acides gras des polyglycérols contient ( pour 100 parties en poids du mélange d'esters d'acides gras des polyglycérols),
de 20 à 65 % en poids d'esters d'acides gras du triglycérol,
de 20 à 50 % en poids d'esters d'acides gras du tétraglycérol, et
de 5 à 40 % en poids d'esters d'acides gras de polyglycérols supérieurs,
et ne contient pas, ou seulement de faibles quantités (moins de 5 % en poids) d'esters d'acides gras du diglycérol,
où le constituant acide gras est constitué d'un ou plusieurs acides gras, choisis parmi les acides gras en C₆-C₁₄, saturés et/ou insaturés, à chaîne droite et/ou ramifiée, contenant moins de 10 % en poids d'acides gras ayant plus de 14 atomes de carbone,
et le rapport HLB du mélange d'esters d'acides gras des polyglycérols est supérieur à 8.

2. Mélange d'esters d'acides gras des polyglycérols selon la revendication 1, caractérisé en ce que le mélange d'esters d'acides gras des polyglycérols contient( pour 100 parties en poids du mélange d'esters d'acides gras des polyglycérols),
de 22 à 32 % en poids d'esters d'acides gras du triglycérol,
de 39 à 49 % en poids d'esters d'acides gras du tétraglycérol et
de 24 à 34 % en poids d'esters d'acides gras de polyglycérols supérieurs,
et ne contient pas, ou seulement de petites quantités (moins de 3 % en poids) d'esters d'acides gras du diglycérol,
où le constituant acide gras est constitué d'un ou plusieurs acides gras choisis parmi les acides gras en C₈ à C₁₂ saturés et/ou insaturés, à chaîne droite et/ou ramifiée, contenant moins de 5 % en poids d'acides gras ayant plus de 14 atomes de carbone,
et le rapport HLB du mélange d'esters d'acides gras des polyglycérols est supérieur à 10.

3. Mélange d'esters d'acides gras des polyglycérols selon les revendications 1 ou 2, caractérisé en ce qu'il comporte de 5 à 20 % en poids d'esters d'acides gras du pentaglycérol.

4. Mélange d'esters d'acides gras des polyglycérols selon l'une ou plusieurs des revendications 1 à 3, caractérisé en ce que le constituant acide gras est constitué de l'acide caprylique, de l'acide caprique, de l'acide laurique, de l'acide undécénoïque, de l'acide 2-éthylhexanoïque et/ou de l'acide gras déricé de l'huile de coprah.

5. Mélange d'esters d'acides gras des polyglycérols selon l'une ou plusieurs des revendications 1 à 4, caractérisé en ce qu'il est constitué, pour plus de 50 % en poids, de mono- et de diesters.

6. Préparation d'additif pour le bain, en particulier préparation pour bain d'huile, comprenant :
un mélange d'esters d'acides gras des polyglycérols constitué de mono-, di- et polyesters de polyglycérols avant un degré de polymérisation de 2 à 8 et d'au moins un acide gras saturé et/ou insaturé,
au moins un constituant ou contenant une huile, choisi parmi les huiles naturelles et synthétiques, minérales, essentielles et grasses, animales et végétales
éventuellement un solvant ou un mélange de solvants, et éventuellement d'autres additifs et auxiliaires, caractérisée en ce qu'elle contient
de 10 à 60 % en poids d'un mélange d'esters d'acides gras des polyglycérols servant d'émulsifiant soluble dans l'eau et/ou de solubilisateur,
de 10 à 60 % en poids d'une huile, d'un mélange d'huiles et/ou d'un constituant huileux à effet cosmétiques et/ou thérapeutique, et
de 0 à 70 % en poids d'eau,
où le mélange d'esters d'acides gras des polyglycérols contient, pour 100 parties en poids du mélange d'esters d'acides gras des polyglycérols,
de 20 à 65 % en poids d'esters d'acides gras du triglycérol,
de 20 à 50 % en poids d'esters d'acides gras du tétraglycérol, et
de 5 à 40 % en poids d'esters d'acides gras de polyglycérols supérieurs,
et ne contient pas, ou seulement de petites quantités (moins de 5 % en poids) d'esters d'acides gras du diglycérol, et le constituant acide gras est constitué d'un ou plusieurs acides gras choisis parmi les acides gras en C₆ à C₁₄ saturés et/ou insaturés, à chaîne droite ou ramifiée, comportant moins de 10 % en poids d'acides gras ayant plus de 14 atomes de carbone.

7. Préparation d'additif pour le bain selon la revendication 6, caractérisée en ce qu'elle contient
de 15 à 50 % en poids d'un mélange d'esters d'acides gras des polyglycérols,
de 15 à 50 % en poids d'une huile, d'un mélange d'huiles et/ou d'un constituant huileux à effet cosmétique et/ou thérapeutique, et
de 0,5 à 60 % en poids d'eau,
où le mélange d'esters d'acides gras des polyglycérols contient, pour 100 parties en poids du mélange d'esters d'acides gras des polyglycérols,
de 22 à 32 % en poids d'esters d'acides gras du triglycérol,
de 39 à 49 % en poids d'esters d'acides gras du tétraglycérol, et
de 24 à 34 % en poids d'esters d'acides gras de polyglycérols supérieurs,
et ne contient pas, ou seulement de petites quantités (moins de 3 % en poids) d'esters d'acides gras du diglycérol, et le constituant acide gras est constitué d'un ou plusieurs acides gras choisis parmi les acides gras en C₈ à C₁₂ saturés et/ou insaturés, à chaîne droite et/ou ramifiée, contenant moins de 5 % en poids d'acides gras ayant plus de 14 atomes de carbone.

8. Procédé de préparation d'un mélange d'esters d'acides gras des polyglycérols, constitué de mono-, di- et polyesters de polyglycérols, avec un degré de polyméri-sation de 2 à 8, et d'au moins un acide gras saturé et/ou insaturé, caractérisé en ce qu'on fait réagir en présence d'au moins un catalyseur, de préférence d'un catalyseur acide, et en dépression, un polyglycérol contenant( pour 100 parties en poids de polyglycérol),
de 20 à 65 % en poids de triglycérol,
de 20 à 50 % en poids de tétraglycérol, et
de 5 à 40 % en poids de polyglycérols supérieurs
et ne contenant pas, ou seulement de petites quantités (moins de 5 % en poids) de diglycérol, avec un ou plusieurs acides gras choisis parmi les acides gras en C₆ à C₁₄ saturés et/ou insaturés, à chaîne droite ou ramifiée, où l'acide gras ou le mélange d'acides gras contient moins de 10 % en poids d'acides gras ayant plus de 14 atomes de carbone, selon un rapport en moles du polyglycérol à l'acide gras ou au mélange d'acides gras de 4:1 à 1:1 et de préférence de 2,5:1 à 1,5:1,
le mélange réactionnel étant d'abord chauffé à une température de 140°C, de préférence de 145°C, et la pression étant réduite à 600 mbar, de préférence à 500 mbar, puis le mélange réactionnel est chauffé, la température étant pilotée selon un programme de températures, dans un intervalle de températures de 140 à 220°C et de préférence de 145 à 190°C, pas à pas ou d'une manière continue, la pression étant abaissée pas à pas ou d'une manière continue, en étant pilotée selon un programme de pression, de 600 à 10 mbar et de préférence de 500 à 20 mbar, l'eau de réaction qui se produit étant chassée en continu par distillation, et, quand l'indice d'acide atteint une valeur < 3, le mélange obtenu d'esters d'acides gras des polyglycérols est refroidi et éventuellement traité et/ou purifié.

9. Procédé selon la revendication 8, caractérisé en ce que le polyglycérol utilisé contient ( pour 100 parties en poids de polyglycérol),
de 22 à 32 % en poids de triglycérol,
de 39 à 49 % en poids de tétraglycérol et
de 24 à 34 % en poids de polyglycérols supérieurs
et ne contient pas, ou seulement de petites quantités (moins de 3 % en poids) de diglycérol.

10. Procédé selon les revendications 8 ou 9, caractérisé en ce qu'on utilise un acide gras ou un mélange d'acides gras choisis parmi les acides gras en C₈ à C₁₂ saturés et/ou insaturés, à chaîne droite et/ou ramifiée, contenant moins de 5 % en poids d'acides gras ayant plus de 14 atomes de carbone.

11. Procédé selon l'une ou plusieurs des revendications 8 à 10, caractérisé en ce qu'on refroidit à une température de 30 à 110°C et de préférence de 60 à 80°C le mélange obtenu d'esters d'acides gras des polyglycérols, puis, par extraction, on le débarrasse partiellement des fractions de polyglycérols n'ayant pas réagi, en ajoutant au mélange d'esters d'acides gras des polyglycérols un solvant ou un mélange de solvants de la chimie organique, puis en extrayant le polyglycérol à l'eau, de préférence dans un étage d'extraction, auquel cas, dans le premier étage d'extraction, on ajoute une quantité en poids correspondant à l'indice d'acide du mélange d'esters d'acides gras des polyglycérols et/ou au moins équivalente à la quantité de catalyseur d'un composé à réaction basique, minéral et/ou organique, et la phase organique restant après l'extraction est débarrassée du solvant organique utilisé et de l'eau résiduelle, par distillation, de préférence par distillation sous vide ou par évaporation sous vide.

12. Procédé selon l'une ou plusieurs des revendications 8 à 11, caractérisé en ce que le solvant ou le mélange de solvants de la chimie organique comporte un pouvoir d'absorption d'eau inférieur à 30 % en poids et de préférence inférieur à 20 % en poids ( pour 100 parties en poids du solvant ou du mélange de solvants de la chimie organique) et/ou forme un mélange azéotropique avec l'eau lors de la distillation ou dans la phase gazeuse.

13. Procédé selon l'une ou plusieurs des revendications 8 à 12, caractérisé en ce qu'on utilise comme solvant de la chimie organique l'acétate d'éthyle, et de préférence l'acétate d'éthyle saturé en eau.

14. Procédé selon l'une ou plusieurs des revendications 8 à 13, caractérisé en ce que, pour neutraliser le catalyseur, on ajoute dans le premier étage d'extraction, au solvant ou au mélange de solvants de la chimie organique et/ou à la phase aqueuse, de l'hydroxyde de sodium et de préférence une solution aqueuse d'hydroxyde de sodium.

15. Procédé selon l'une ou plusieurs des revendications 8 à 14, caractérisé en ce que, pour neutraliser le catalyseur, on ajoute dans le premier étage d'extraction au solvant ou au mélange de solvants de la chimie organique et/ou à la phase aqueuse des carbonates alcalins, de préférence du carbonate de sodium et/ou du carbonate de potassium, et/ou un échangeur d'ions basique.

16. Procédé selon l'une ou plusieurs des revendications 8 à 15, caractérisé en ce que le mélange d'esters d'acides gras des polyglycérols est abandonné, avant une post-transformation et/ou une purification, pendant plus de 0,5 heure et de préférence pendant 1 à 10 heures, et éventuellement on sépare les fractions précipitées de polyglycérols.

17. Procédé selon l'une ou plusieurs des revendications 8 à 16, caractérisé en ce que le chauffage le mélange réactionnel s'effectue dans l'intervalle de températures de 140 à 220°C et de préférence de 145 à 190°C, et que la diminution de la pression de 600 à 10 mbar et de préférence de 500 à 20 mbar s'effectue dans un laps de temps de 2 à 6 heures et de préférence de 3 à 4 heures.

18. Procédé selon l'une ou plusieurs des revendications 8 à 17, caractérisé en ce que le chauffage du mélange réactionnel dans l'intervalle de températures de 140 à 220°C et de préférence de 145 à 190°C s'effectue pas à pas en 3 à 6 étapes et de préférence en 4 à 5 étapes.

19. Procédé selon l'une ou plusieurs des revendications 8 à 18, caractérisé en ce que la diminution de pression de 600 à 10 mbar et de préférence de 500 à 20 mbar s'effectue pas à pas en 3 à 6 étapes et de préférence en 4 à 5 étapes.

20. Procédé selon l'une ou plusieurs des revendications 8 à 19, caractérisé en ce que le catalyseur acide, de préférence un composé contenant des groupes acide sulfonique, est utilisé en combinaison avec un composé acide à effet réducteur, de préférence l'acide hypophosphoreux.

21. Procédé selon l'une ou plusieurs des revendications 8 à 20, caractérisé en ce que le mélange réactionnel est mis à réagir en présence d'un gaz inerte, de préférence l'azote.

22. Utilisation du mélange d'esters d'acides gras des polyglycérols selon l'une ou plusieurs des revendications 1 à 5 comme émulsifiant, solubilisateur, dispersant, mouillant et/ou regraissant dans des préparations cosmétiques, pharmaceutiques ou utilisées en technique chimique.

23. Utilisation du mélange d'esters d'acides gras des polyglycérols selon la revendication 22 pour solubiliser et/ou émulsionner des huiles naturelles ou synthétiques, essentielles, minérales, grasses, animales ou végétales.

24. Utilisation du mélange d'esters d'acides gras des polyglycérols selon la revendication 22 comme émulsifiant dans des huiles de forage ou des fluides de forage.

25. Utilisation du mélange d'esters d'acides gras des polyglycérols selon la revendication 22 comme mouillant et/ou dispersant dans des détergents techniques.

26. Utilisation du mélange d'esters d'acides gras des polyglycérols selon la revendication 22 comme émulsifiant et/ou dispersant dans des préparations colorantes, de préférence dans des colorants de dispersion.

27. Utilisation du mélange d'esters d'acides gras des polyglycérols selon l'une ou plusieurs des revendications 1 à 5 comme additif pour les soins de la peau et/ou produit lavant, détergent ou nettoyant pour les soins corporels.

28. Utilisation du mélange d'esters d'acides gras des polyglycérols selon l'une ou plusieurs des revendications 1 à 5 comme lubrifiant soluble dans l'eau dans des préparations utilisées en technique chimique.
